(19) Europäisches Patentamt
European Patent Office
Office européen des brevets

(11) Publication number: 0 080 229
B1

(12) # EUROPEAN PATENT SPECIFICATION

(45) Date of publication of patent specification: 13.03.85

(51) Int. Cl.$^4$: **C 07 C 153/11**, C 07 H 17/08, C 07 C 101/24, C 07 C 103/19, A 61 K 31/65, A 61 K 31/615, A 61 K 31/195, A 61 K 31/71

(21) Application number: 82201443.7

(22) Date of filing: 12.11.82

(54) **Salicylic derivatives of N-acetylcysteine.**

(30) Priority: 20.11.81 IT 2519881

(43) Date of publication of application: 01.06.83 Bulletin 83/22

(45) Publication of the grant of the patent: 13.03.85 Bulletin 85/11

(84) *Designated Contracting States:* AT BE CH DE FR GB LI LU NL

(58) References cited: FR-M- 4 619

(73) Proprietor: Pietro Isnardi & C. Spa
Via XXV Aprile 69
I-18100 Oneglia Imperia (IT)

(72) Inventor: Ponchiroli, Osvaldo
Via A. Sciesa 18
I-20017 Rho Milano (IT)

(74) Representative: Dragotti, Gianfranco
SAIC BREVETTI S.a.s. di Ing. G. Dragotti & C. Via Spontini, 1
I-20131 Milano (IT)

**The file contains technical information submitted after the application was filed and not included in this specification**

EP 0 080 229 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to the thio-ester of N-acetylcysteine with the salicylic acid and the pharmaceutically acceptable and non toxic organic and inorganic salts thereof, of general formula:

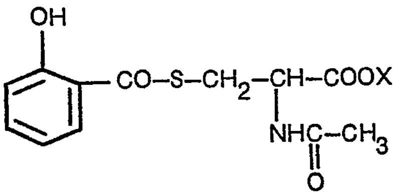

wherein X is hydrogen, an alkali metal, an earth-alkali metal, or the radical of an organic base, particularly a basic aminoacid or a basic antibiotic.

It has been discovered, as it is confirmed by the preliminary searches on the pharmacological activities, that there not only are maintained the specific properties of the salicylic acid and of the N-acetylcysteine, i.e. the antipyretic, anti-inflammatory and analgesic activities for the former and the mucolytic activity for the latter, but these properties are also surprisingly improved in the compounds of this invention, the latter being thus suitable for use as expectorant, mucolytic, anti-pyretic and anti-inflammatory drugs.

As stated above the present invention also relates to the pharmaceutically non toxic, organic and inorganic salts of the thio-ester of N-acetylcysteine with the salicylic acid.

Among the inorganic bases which are useful for this purpose, those of alkali and earth-alkali metals are contemplated, whereas among the organic ones basic aminoacids, such as arginine and lysine, and the basic antibiotics, such as erythromycin, propionylerythromycin, neomycin, mechlocycline are of particular importance, the specific properties and activities of which being thus exploited.

The method being also and object of the present invention essential comprises the following steps:

a) preparing the chloride salt of the acetyl salicylic acid by means of a chlorinating agent per se known, or preparing the mixed anhydride of the acetylsalicylic acid with ethyl chloroformate;

b) reacting N-acetylcysteine, at low temperature in an aqueous and esteric medium and in presence of an organic or inorganic base acting as acid acceptor with the chloride of the acetylsalicylic acid or the mixed anhydride of the acetylsalicylic acid;

c) isolating of pure deacetylated thioester and possibly converting the same into the corresponding organic or inorganic salts in an aqueous, alcoholic or ketonic medium.

According to the preferred embodiment of the invention there is carried out the chlorination of acetylsalicylic acid by means of thionyl chloride, and by refluxing the reaction mass, and the esterification, carried out in water and at temperature ranging from 1°C to 5°C.

Among the esteric solvents for the reaction of the above mentioned process there are for example included ethylacetate, isopropyl acetate, and so on.

An alkali metal hydrate or a tertiary amine, such as for example triethylamine, can be used as an organic or inorganic base having acid acceptor activity.

As regards the step (c), it should be pointed out that the reaction mixture will partly consist of an already deacetylated product and partly of a still acetylated ester. In such a case, before insolation is carried out, the deacetylation of the acetylated portion is made, for example by reaction with an organic base.

It is finally to be noted that in the process of the invention, by N-acetylcysteine there is meant the levoratatory form thereof, i.e. the usually available one. This meaning should not in any case be intended as an improper limitation of the invention.

Additionally the reaction temperature of the step (3) does not exceed 5°C.

The invention will be now illustrated in non limiting sense by the following examples.

Example 1

a) Chlorination of the acetylsalicylic acid

100 g of acetylsalicylic acid are placed into a 3 necked flask equipped with a stirrer, a smoke trap, and a reflux condenser, together with 25 ml of anhydrous chloroform and with 250 ml of thionyl chloride. The mass is slowly heated under stirring and after about 40 minutes of refluxing a perfect solution having a pale yellow colour is obtained.

Upon the evolving of gaseous $SO_2$ and HCl is completed, the reaction mixture is cooled under stirring to room temperature, and then concentrated under vacuum to eliminate the excess solvent and thionylchloride, thus obtaining a straw-coloured liquid (110,3 g).

b) Mixed anhydride of acetylsalicylic acid and ethyl chloroformate

18 g of acetylsalicylic acid are charged into a 3 necked flask, equipped with a stirrer and with a separatory funnel, together with 180 ml of anhydrous benzene and 11 g of ethyl chloroformate.

The mass under stirring is cooled to a temperature of about 0°C, then 10 g of triethylamine are slowly added, thus giving place to a heterogeneous reaction mass containing a precipitate which is filtered under vacuum, and a solution of the anhydride is obtained which, when concentrated, gives 25 g of a yellow limpid oil.

c) Preparation of salicyloylthio-N-acetylcysteine

A 1 l flask, equipped with a stirrer and with

an outer refrigerating bath, is charged with 102.8 g of sodium salt of N-acetylcysteine and 700 ml of water.

The solution is cooled to a temperature of between 1 and 5°C, and a solution of 22,2 g of NaOH, dissolved in 120 ml of water, and of acetylsalicyloylchloride is slowly added.

The addition is adjusted so that the temperature does not exceed 5°C and it is completed within about 1 h.

Upon the addition is terminated, the temperature of the reaction mixture, the latter being maintained under stirring, does spontaneously increase to 15°C; then the reaction mixture is maintained at rest still under stirring for about 1 h.

The reaction mixture is slowly acidified and the finished product is extracted with 700 ml of methylisobutyl ketone.

The ketonic phase is separated, washed with water, dried, filtered on charcoal, concentrated and there are obtained 180 g of dense oil, comprising both the desired ester and a not negligible amount of acetylated compound, i.e. the acetylsalicylic ester.

32.5 g of this product are reacted with 0,1 mole of an organic base, such as DL-lysine, freshly obtained as a 50% aqueous solution, in admixture with methanol and isopropanol. The thus obtained salt (with a yield of 75% of the theoretical value) is in forme of white crystals having m.p. 110—112°C.

20 g of this product are dissolved into 100 ml of distilled water and made acidic with 0,5 N hydrogen chloride up to pH 3.5 under stirring.

The salicyloylthio N-acetylcysteine acid is obtained as white crystals which after filtration, washing and drying gives place to a yield of 10 g with a m.p. 173—175°C.

1 g of crude salicyloylthio N-acetylcysteine acid can be crystallized with 10ml of a solution water/ethanol (50/50) thus obtaining a product of m.p. 192—195°C, chromatographically pure with a yield of 80%.

The product, which has the formula (1), in which X=H, and molecular weight 283.31, appears as non hygroscopic white coloured microcrystalline powder, little soluble in cold water, partly soluble is alcohols and non soluble in chloroform and in ethers.

From the chromatographic analysis on silica gel using ethylacetate/ethanol/acetic acid (35:20:2) as eluant, U.V. light as detector and a $FeCl_3$ 8% solution, an Rf value of 0.54 is found.

Example 2

Sodic salt of salicyloylthio-N-acetylcysteine

To a suspension of 1 mole of salicyloylthio-N-acetylcysteine in an equilibrium mixture of dioxane and water the stoichiometric amount of $NaHCO_3$ is added, the reaction temperature being maintained at a value not higher than +10°C. The thus obtained solution is liophilized to give the pure crystalline form of the sodic salt.

Example 3

Erythromycin salt of salicyloylthio-N-acetylcysteine

Erythromycin base (13 g) is reacted with 5 g of salicyloylthio-N-acetyl-cysteine in 10 ml of ethyl-acetate.

A homogeneous phase is obtained, then 4 mls of water are added and a white crystalline product is formed which after 48 h is filtered, washed and dried, whereby the desired salt is obtained with a yield of 67% (of the theoretical value) having a m.p. 83—87°C.

Example 4

Monopropionate erythromycin salt of salicyloyl-thio-N-acetyl-cysteine

Erythromycin monopropionate (14 g) is reacted with 5 g of salicyloylthio-N-acetylcysteine in 100 ml of anhydrous cyclohexane.

There is thus obtained a suspension, which is stirred and slowly added with 6 mls of methanol and a transformation of the crystalline characteristics takes place.

The stirring is contained during 2 h; then filtration, washing and drying are carried out. The salt is obtained with a yield of 95% of the theoretical value and it is in the form of a white powder with a transformation point at 100°C.

Example 5

0.1 moles of mechlocycline base are stirred with a mixture of water (30 ml) and ethanol (30 ml) and then 0,1 moles of salicyloyl-N-acetyl-cysteine acid are added. A homogeneous phase is obtained.

Upon the solution is made completely homogeneous, isopropylic acid (20 ml) is slowly added.

A yellow product precipitates.

After filtration 5,6 g of salt are obtained having m.p. 85—90°C (with decomposition).

The derivatives of the invention have been subjected to toxicological and pharmacological tests, and hereinafter only the data relating to lysinate of salicyloylthio-N-cysteine are reported without it having limiting purposes.

There were found in the mouse the following $LD_{50}$ values:

$LD_{50}$ (oral)>4000 mg/kg.

$LD_{50}$ (intraperitoneal route)>3000 mg/kg.

In the rat the following $LD_{50}$ values were found:

$LD_{50}$ (oral)>4000 mg/kg.

$LD_{50}$ (intraperitoneal route) 1460±235 mg/kg.

In the carrageenin induced oedema test, an $ED_{33}$ value of 235 mg/kg was detected.

In the arthritis test induced by Freund adjuvant, was found an $ED_{33}$ value of 131 mg/kg.

In the test on the analgesic activity inhibition

test of stretching or writhing induced by acetic acid an ED=210 mg/kg was found.

In the test of mucolitic activity in vitro, the viscosity of mucus was reduced by about 8% by a concentration of the tested compound of 0.05 moles.

In the test of antipyretic activity an $ED_{75}$ value of 427 mg/kg was found.

The derivatives of the present invention are used to prepare pharmaceutical compositions suitable for the desired administration route, together with the usual carriers, excipients and fillers and by means of the usual pharmaceutical manufacturing technologies, the foreseen therapeutical dosages being of being 0.3 and 1 g.

As regards the specific case of the salt with mechlocycline, the dermatological activity of which is known, therapeutical activity of the antibiotic becomes improved and completed.

## Claims

1. Salicylic derivatives of N-acetylcysteine of general formula:

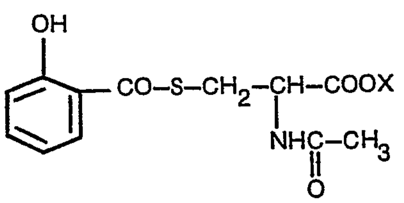

wherein X is selected in the group consisting of hydrogen alkali metals, earth alkali metals, and the radicals of organic bases.

2. Salicylic derivatives according to claim 1, characterized in that said organic base is a basic aminoacid.

3. Salicylic derivatives according to claim 2, characterized in that said basic aminoacid is selected among lysine and arginine.

4. Salicylic derivatives according to claim 1, characterized in that said organic base is a basic antibiotic.

5. Salicylic derivatives according to claim 4, characterized in that said basic antibiotic is selected among erythromycin, propionylerythromycin, neomycin and mechlocycline.

6. Salicyclic derivatives according to claim 1, characterized in that said alkali metal is sodium.

7. A process for the preparation of the salicylic derivatives of claim 1, characterized by the steps of:

a) preparing a reactive derivative of acetylsalicylic acid, of the group comprising chloride and the mixed anhydride with ethyl chloroformate;

b) reacting, at low temperature, with N-acetylcysteine, in presence of an organic or inorganic base acting as an acceptor of acids;

c) isolating the resulting thioester and possibly converting the same into the corresponding organic or inorganic salt in an aqueous, alcoholic or ketonic medium.

8. A process according to claim 7, characterized in that said reactive derivative is the chloride of acetyl salicylic acid and is prepared by refluxing said acetylsalicylic acid with thionyl chloride.

9. A process according to claim 7, characterized in that said reactive derivative is the mixed anhydride, and is prepared by reacting acetylsalicylic acid and ethyl chloroformate, at a low temperature and an organic solvent.

10. A process according to claim 7, characterized in that said reaction between said reactive derivative and said N-acetylcysteine is carried out in water and at a temperature ranging from 1°C to 5°C in presence of an alkaline hydroxide.

11. A process according to claim 7, characterized in that said alkaline hydroxide is sodium hydroxide.

12. A process according to claim 7, characterized in that said reaction between said reactive derivative and N-acetylcysteine is carried out in an esteric medium, at a temperature of between 1°C and 5°C, and in the presence of an organic base.

13. A process according to claim 12, characterized in that said organic base is a tertiary amine.

14. A process according to claims 10 or 12, characterized in that the reaction product is treated with an organic base to complete the deacetylation, and the deacetylated product undergoes acid hydrolysis, thus obtaining salicyloylthio-N-acetyl-cysteine acid.

15. A process according to claim 7, characterized in that said possible conversion is carried out by reacting the salicyloylthio-N-acetylcysteine acid with an organic or inorganic base.

16. A process according to claim 15, characterized in that said inorganic base is sodium carbonate and the reaction is carried out at a temperature not exceeding 10°C in an equilibrium mixture of dioxane and water.

17. A process according to claim 15, characterized in that said organic base is (L) or (DL)-lysine in an aqueous solution, and the reaction takes place in a mixture of methanol and isopropanol.

18. A process according to claim 15, characterized in that said organic base is selected among the group consisting of erythromycin, erythromycin propionate and mechlocycline.

19. Pharmaceutical composition having antipyretic, anti-inflammatory, analgesic and mucolitic activity, characterized by containing, as active component, a salicylic derivative of the claim 1.

20. Pharmaceutical composition according to claim 19, in suitable form for oral, parenteral, rectal and topical administration.

21. Pharmaceutical composition in suitable form for topical administration, having dermatological activity, characterized by containing, as active component, the salt with mechlocycline of the salicylic derivative according to claim 1.

**Revendications**

1. Dérivés salicyliques de la N-acétyl-cystéine de formule générale:

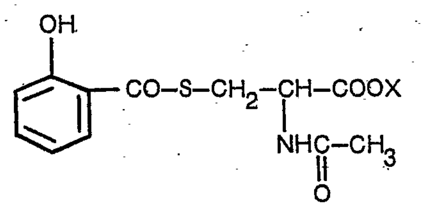

où X est choisi dans le groupe comprenant l'hydrogène, les métaux alcalins, les métaux alcalino-terreux, et les radicaux de bases organiques.

2. Dérivés salicyliques selon la revendication 1, caractérisés en ce que ladite base organique est un amino-acide basique.

3. Dérivés salicyliques selon la revendication 2, caractérisés en ce que ledit amino-acide basique est choisi parmi la lysine et l'arginine.

4. Dérivés salicyliques selon la revendication 1, caractérisés en ce que ladite base organique est une antibiotique basique.

5. Dérivés salicyliques selon la revendication 4, caractérisés en ce que ledit antibiotique basique est choisi parmi l'érythromycine, la proprionylérythromycine, la néomycine et la méchlocycline.

6. Dérivés salicyliques selon la revendication 1, caractérisés en ce que ledit métal alcalin est du sodium.

7. Procédé pour la préparation de dérivés salicyliques selon la revendication 1, caractérisé par les étapes consistant à:

a) préparer un dérive réactif de l'acide acétylsalicylique, du groupe comprenant le chlorure et l'anhydride mélangés avec du chloroformate d'éthyle;

b) faire dérouler la réaction à basse température avec de la N-acétylcystéine, en présence d'une base organique ou inorganique agissant en tant qu'accepteur d'acides;

c) isoler le thioesther résultant et éventuellement convertir ce dernier en sel organique ou inorganique correspondant dans un milieu aqueux, alcoolique ou cétonique.

8. Procédé selon la revendication 7, caractérisé en ce que ledit dérivé réactif est le chlorure de l'acide acétylsalicylique et est préparé en refluxant ledit acide acétylsalicylique avec du chlorure de thionyle.

9. Procédé selon la revendication 7, caractérisé en ce que ledit dérivé réactif est l'anhydride mélangé, préparé en faisant réagir l'acide acétylsalicylique et le chloroformate d'éthyle à basse température et dans un solvant organique.

10. Procédé selon la revendication 7, caractérisé en ce que ladite réaction entre ledit dérivé réactif et ladite N-acétylcystéine est réalisée dans de l'eau et à une température comprise entre 1°C et 5°C, en présence d'un hydroxyde alcalin.

11. Procédé selon la revendication 7, caractérisé en ce que ledit hydroxyde alcalin est de l'hydroxyde de sodium.

12. Procédé selon la revendication 7, caractérisé en ce que ladite réaction entre ledit dérivé réactif et la N-acétylcystéine est réalisée dans un milieu estérique, à une température comprise entre 1°C et 5°C et en présence d'une base organique.

13. Procédé selon la revendication 12, caractérisé en ce que ladite base organique est une amine tertiaire.

14. Procédé selon la revendication 10 ou 12, caractérisé en ce que le produit de la réaction est traité avec une base organique pour terminer la désacétylation, et le produit désacétylé est soumis à une hydrolyse acide, pour obtenir l'acide salicyloylthio-N-acétylcystéinique.

15. Procédé selon la revendication 7, caractérisé en ce que ladite conversion possible ets réalisée en faisant réagir l'acide salicyloylthio-N-acétylcystéinique avec une base organique ou inorganique.

16. Procédé selon la revendication 15, caractérisé en ce que ladite base inorganique est du carbonate de sodium et la réaction est réalisee à une température qui ne dépasse pas 10°C, dans un mélange en équilibre de dioxane et d'eau.

17. Procédé selon la revendication 15, caractérisé en ce que ladite base organique est de la (L)-lysine ou de la (DL)-lysine en solution aqueuse, et la réaction a lieu dans un mélange de méthanol et d'isopropanol.

18. Procédé selon la revendication 15, caractérisé en ce que ladite base organique est choisie parmi le groupe comprenant l'érythromycine, l'érythromycine-propionate et la méchlocycline.

19. Composition pharmaceutique présentant une activité antipyrétique, antiinflammatoire, analgésique et mucolitique, caractérisée en ce que elle contient, en tant que composant actif, un dérivé salicylique selon la revendication 1.

20. Composition pharmaceutique selon la revendication 19, se présentant sour une forme convenant à une administration orale, parantérale, rectale et topique.

21. Composition pharmaceutique se présentant sour une forme convenant à une administration topique, présentant une activité dermatoloqique, caractérisée en ce qu'elle contient, en tant que composant actif, le sel avec la méchlocycline du dérivé salicylique selon la revendication 1.

**Patentansprüche**

1. Salicylderivative von N-Acetyl-Cystein der allgemeinen Formel

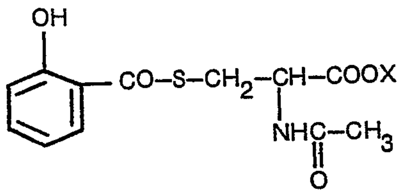

worin X in der Gruppe ausgewählt ist, die aus den Wasserstoffalkalimetallen, Erdalkalimetallen und den Radikalen organischen Basen besteht.

2. Salicylderivative nach Anspruch 1, dadurch gekennzeichnet daß die gennante organische Base einer basische Aminosäure ist.

3. Salicylderivative nach Anspruch 2, dadurch gekennzeichnet daß die gennante basische Aminosäure unter Lysin und Arginin augsgewählt ist.

4. Salicylderivative nach Anspruch 1, dadurch gekennzeichnet, daß die genannte organische Base ein basisches Antibiotikum ist.

5. Salicylderivative nach Anspruch 4, dadurch gekennzeichnet, daß das gennante basische Antibiotikum unter Erythromycin, Propionylerythromycin, Neomycin und Mechlocyclin ausgewählt ist.

6. Salicylderivative nach Anspruch 1, dadurch gekennzeichnet, daß das gennante Alkalimetall Natrium ist.

7. Verfahren zur Herstellung der Salicylderivative nach Anspruch 1, gekennzeichnet durch die folgenden Schritte:

a) Zubereitung eines reaktions fähigen Acetylsalicylsäurederivats der Gruppe unfassend Chlorid und das mit Äthylchloroformat gemischte Anhydrid;

b) Umsetzung it N-Acetyl-Cystein bei niedriger Temperatur und in Gegenwart einer organischen oder anorganischen Base, die als Säureakzeptor wirkt;

c) Isolierung des sich ergebenden Thioesters und gegebenenfalls Unwandlung desselben in das entsprechende organische oder anorganische Salz in einem wassrigen, alkoholischen oder ketonischen Medium.

8. Verfahren nach Anspruch 7, dadurch gekennzeichnet, daß das genannte reaktionsfähige Derivat Acetylsalicylsäurechlorid ist und durch Rückflußbehandlung der genannten Acetylsalicylsäure mit Thionylchlorid zubereitet wird.

9. Verfahren nach Anspruch 7, dadurch gekennzeichnet, daß das genannte reaktionsfähige Derivat das gemischte Anhydrid ist und durch Umsetzung von Acetylsalicylsäure und Athylchloroformat bei niedriger Temperatur in einem organischen Lösungsmittel zubereitet wird.

10. Verfahren nach Anspruch 7, dadurch gekennzeichnet, daß die genannte Umsetzung zwischen dem genannten reaktionsfähigen Derivat und dem genannten N-Acetyl-Cystein in Wasser und innerhalb eines Temperaturbereiches von 1°C bis 5°C in Gegenwart von einem alkalinen Hydroxyd durchgeführt wird.

11. Verfahren nach Anspruch 7, dadurch gekennzeichnet, daß das gennante alkaline Hydroxyd Natriumhydroxyd ist.

12. Verfahren nach Anspruch 7, dadurch gekennzeichnet, daß die genannte Umsetzung zwischen dem genannten reaktionsfähigen Derivat und N-Acetyl-Cystein in einem esterischen Medium bei einer Temperatur zwischen 1°C und 5°C und in Gegemwart einer organischen Base durchgeführt wird.

13. Verfahren nach Anspruch 12, dadurch gekennzeichnet, daß die genannte organischen Base ein tertiares Amin ist.

14. Verfahren nach Anspruch 10 oder 12, dadurch gekennzeichnet, daß das Umsetzungsprodukt zur Vervollständigung der Deacetylation mit einer organischen Base behandelt wird und daß das deacetylierte Produkt einer Säurehydrolyse unterworfen wird, so daß Salicyloylthio-N-Acetyl-Cysteinsäure erhalten wird.

15. Verfahren nach Anspruch 7, dadurch gekennzeichnet, daß die etweige Umwandlung durch Umsetzung der Salicyloylthio-N-Acetyl-Cysteinsäure mit einer organischen oder anorganischen Base durchgeführt wird.

16. Verfahren nach Anspruch 15, dadurch gekennzeichnet, daß die genannte anorganische Base Natriumcarbonat ist und die Umsetzung bei einer, 10°C nich übersteigenden Temperatur in einer ausgeglichenen Mischung von Dioxan und Wasser durchgeführt wird.

17. Verfahren nach Anspruch 15, dadurch gekennzeichnet, daß die genannte organische Base (L)- oder (DL)-Lysin in wässriger Lösung ist und die Umsetzung in einer Mischung von Methanol und Isopropanol stattfindet.

18, Verfahren nach Anspruch 15, dadurch gekennzeichnet, daß die genannte organische Base in der Gruppe ausgewählt ist, due aus Erythromycin, Erythromycinpropionate und Mechlocyclin besteht.

19. Pharmazeutische Zusammensetzung mit antipyretischer, antiinflammatorischer, analgesischer und mucolitischer Wirksamkeit, gekennzeichnet durch den Gehalt als Wirkbestandteil eines Salicylderivats nach Anspruch 1.

20. Pharmazeutische Zusammensetzung nach Anspruch 19, geeignet für orale, parenterale, rectale und topische Verabreichung.

21. Pharmazeutische Zusammensetzung in für topische Verabreichung geeigneter Form mit dermatologischer Wirksamkeit, gekennzeichnet durch einen Gehalt als Wirkbestandteil des Salzes des Salicylderivatives nach Anspruch 1 mit Mechlocyclin.